Europäisches Patentamt

European Patent Office

Office européen des brevets

(11) Veröffentlichungsnummer: **0 532 981 A1**

## EUROPÄISCHE PATENTANMELDUNG

(21) Anmeldenummer: **92115001.7**

(22) Anmeldetag: **02.09.92**

(51) Int. Cl.5: **A61B 17/22**, A61B 6/00, A61B 6/12

(30) Priorität: **16.09.91 DE 4130761**

(43) Veröffentlichungstag der Anmeldung:
**24.03.93 Patentblatt 93/12**

(84) Benannte Vertragsstaaten:
**DE FR GB**

(71) Anmelder: **SIEMENS AKTIENGESELLSCHAFT**
**Wittelsbacherplatz 2**
**W-8000 München 2(DE)**

(72) Erfinder: **Bock, Hans-Christian, Dipl.-Ing.(FH)**
**Siedlung 9a**
**W-8520 Uttenreuth(DE)**
Erfinder: **Matura, Eike, Dipl.-Ing.(FH)**
**Weiherstrasse 11**
**W-8520 Erlangen(DE)**

(54) **Gerät zur Behandlung eines Lebewesens mit akustischen Wellen.**

(57) Die Erfindung betrifft ein Gerät zur Behandlung eines Lebewesens mit akustischen Wellen, aufweisend eine Quelle (10) akustischer Wellen und eine Röntgen-Ortungseinrichtung mit einem Röntgenstrahler (2) und einem Strahlenempfänger (3), welche einander gegenüberliegend an einem C-Bogen (1) angebracht sind, der entlang seines Umfanges um seine Mittelachse (M) verstellbar in einem Halter (7) aufgenommen ist. Dabei ist vorgesehen, daß die Quelle (10) akustischer Wellen derart entlang des Umfanges des C-Bogens (1) verstellbar ist, daß die akustische Achse (A) der Quelle (10) akustischer Wellen stets die Mittelachse (M) des C-Bogens (1) schneidet.

EP 0 532 981 A1

Die Erfindung betrifft ein Gerät zur Behandlung eines Lebewesens mit akustischen Wellen, aufweisend eine Quelle akustischer Wellen und eine Röntgen-Ortungseinrichtung mit einem Röntgenstrahler und einem Strahlenempfänger, welche einander gegenüberliegend an einem C-Bogen angebracht sind, der entlang seines Umfanges um seine Mittelachse verstellbar in einem Halter aufgenommen ist.

Derartige Geräte, die akustische Wellen in Form von Druckimpulsen, insbesondere Stoßwellen, erzeugen, werden zur Behandlung von Steinleiden (Lithiasis) oder pathologischen Gewebeveränderungen eingesetzt. Aus der US-PS 4 979 501 ist es außerdem bekannt, ein derartiges Gerät zur Behandlung von Knochenleiden einzusetzen. Dabei ist die Quelle akustischer Wellen gemeinsam mit dem C-Bogen der Röntgen-Ortungseinrichtung an einem Träger angebracht, der geradlinig verstellbar ist. Der C-Bogen ist entlang seines Umfanges um einen Winkel von ± 30° verstellbar, der ausreicht, um, so wie dies für Ortungszwecke bekanntermaßen erforderlich ist, ein zu behandelndes Körperteil, beispielsweise eine Extremität, unter unterschiedlichen Winkeln mit Röntgenstrahlung durchstrahlen zu können. Dagegen macht sich der Umstand, daß die Quelle akustischer Wellen nur geradlinig verstellbar ist, insbesondere bei der Behandlung von Knochenleiden im Bereich der Extremitäten nachteilig bemerkbar, da der Patient bzw. die zu behandelnde Extremität häufig umgelagert werden muß, um eine Position relativ zu der Quelle akustischer Wellen zu gewährleisten, die sicherstellt, daß die akustischen Wellen ungehindert zu einem zu behandelnden Bereich gelangen können. Das Erfordernis der ständigen Umlagerung wird sowohl von dem Patienten, der dabei unter Umständen Schmerzen leidet, als auch von dem medizinischen Personal wegen der damit verbundenen körperlichen Anstrengungen als unangenehm empfunden.

Der Erfindung liegt die Aufgabe zugrunde, ein Gerät der eingangs genannten Art so auszubilden, daß auf einfache Weise, insbesondere ohne Umlagerung des Patienten bzw. einer zu behandelnden Extremität, sichergestellt ist, daß die akustischen Wellen zu dem jeweils zu behandelnden Bereich gelangen können.

Nach der Erfindung wird diese Aufgabe gelöst durch ein Gerät zur Behandlung eines Lebewesens mit akustischen Wellen, aufweisend eine Röntgen-Ortungseinheit mit einem Röntgenstrahler und einem Strahlenempfänger, welche einander gegenüberliegend an einem C-Bogen angebracht sind, der entlang seines Umfanges um seine Mittelachse verstellbar in einem Halter aufgenommen ist, und eine Quelle akustischer Wellen, die derart entlang des Umfanges des C-Bogens verstellbar ist, daß die akustische Achse der Quelle akustischer Wellen stets die Mittelachse des C-Bogens schneidet. Infolge des Umstandes, daß die Quelle akustischer Wellen entlang des Umfanges des C-Bogens verstellbar ist, ist es also in einer Vielzahl von Fällen möglich, die Quelle akustischer Wellen und den Körper des Patienten bzw. eine zu behandelnde Extremität relativ zueinander in der erforderlichen Weise auszurichten, ohne daß es einer Umlagerung bedarf. Dabei besteht in vorteilhafter Weise die Möglichkeit, die Quelle akustischer Wellen unabhängig von der Röntgen-Ortungseinrichtung oder gleichzeitig mit dieser zu verstellen. Infolge des Umstandes, daß die akustische Achse der Quelle akustischer Wellen stets die Mittelachse des C-Bogens schneidet, ist in vorteilhafter Weise eine definierte räumliche Zuordnung der Quelle akustischer Wellen relativ zu der Röntgen-Ortungseinrichtung gegeben.

Eine weitere Verbesserung der Situation läßt sich erreichen, wenn gemäß einer Variante der Erfindung die Summe des jenigen Winkels, um den der C-Bogen entlang seines Umfanges in dem Halter verstellbar ist, und desjenigen Winkels, um den die Quelle akustischer Wellen entlang des Umfanges des C-Bogens verstellbar ist, wenigstens gleich 360° ist. In diesem Falle kann nämlich die Quelle akustischer Wellen auf einer vollständigen Kreisbahn um den Patienten bzw. eine zu behandelnde Extremität herumbewegt werden. Es sind also bezogen auf die Mittelachse des C-Bogens beliebige Winkelstellungen des Patienten bzw. einer zu behandelnden Extremität und der Quelle akustischer Wellen relativ zueinander möglich, ohne daß es einer Umlagerung bedarf.

Wenn die Quelle akustischer Wellen fokussierte akustische Wellen abgibt, ist gemäß einer Variante der Erfindung vorgesehen, daß die Fokuszone der akustischen Wellen wenigstens während der Beaufschlagung eines zu behandelnden Bereiches mit akustischer Wellen auf der Mittelachse des C-Bogens liegt. Hierdurch ist eine definierte Lage der Fokuszone relativ zu der Röntgen-Ortungseinrichtung sichergestellt. Um vor und nach der Behandlung günstigere Platzverhältnisse zur Verfügung zu haben, kann vorgesehen sein, daß die Quelle akustischer Wellen, beispielsweise in Richtung ihrer akustischen Achse, von der Mittelachse des C-Bogens weg verstellt werden kann.

Um die Lage des Röntgenstrahlers und des Röntgenbildverstärkers der Röntgen-Ortungseinrichtung den jeweiligen Bedürfnissen anpassen zu können, ist vorgesehen, daß der C-Bogen um eine Achse, die die Mittelachse des C-Bogens vorzugsweise in dem gleichen Punkt wie der Zentralstrahl der Röntgen-Ortungseinrichtung und vorzugsweise im rechten Winkel schneidet, um einen Winkel von wenigstens 180° schwenkbar ist. Es ist dann sozusagen ein Platztausch von Röntgenstrahler und Röntgenbildverstärker möglich.

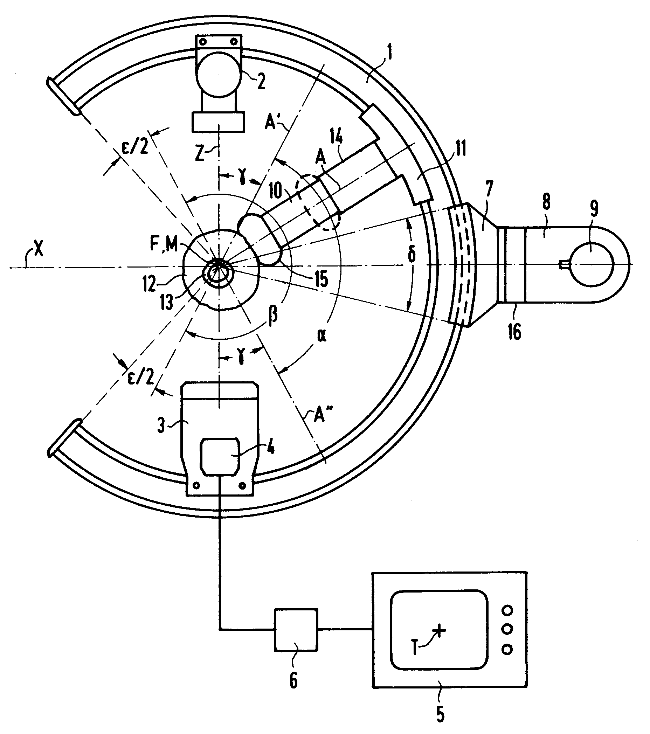

Ein Ausführungsbeispiel der Erfindung, und zwar ein Gerät zur Behandlung von Knochenleiden, ist in der einzigen Figur der beigefügten Zeichnung dargestellt.

Das erfindungsgemäße Gerät weist einen kreisförmig gekrümmten C-Bogen 1 auf, an dem einander diametral gegenüberliegend ein Röntgenstrahler 2 und als Strahlenempfänger ein Röntgenbildverstärker 3 angeordnet sind. Der strichliert angedeutete Zentralstrahl Z des von dem Röntgenstrahler 2 ausgehenden Röntgenstrahlenbündels schneidet die Mittelachse M des C-Bogens 1 und trifft mittig auf dem Eingangsleuchtschirm des Röntgenbildverstärkers 3 auf. Das Bild des Ausgangsleuchtschirmes des Röntgenbildverstärkers 3 wird mittels einer in diesen integrierten Fernsehkamera 4 aufgenommen und auf einem Fernsehmonitor 5 dargestellt. Zwischen die Fernsehkamera 4 und den Fernsehmonitor 5 ist ein Markengenerator 6 geschaltet, der in das auf dem Fernsehmonitor 5 dargestellte Röntgenbild eine Marke T einblendet, deren Lage dem Schnittpunkt des Zentralstrahles Z des Röntgenstrahlenbündels mit dem Eingangsleuchtschirm des Röntgenbildverstärkers 3 entspricht. Der Röntgenstrahler 2 steht mit einer nicht dargestellten, an sich bekannten elektrischen Generatoreinrichtung in Verbindung, die ihn mit den erforderlichen Spannungen versorgt.

Der C-Bogen 1 weist einen doppel-T-förmigen Querschnitt auf. Sein äußerer Flansch ist in einem Halteschuh 7 derart aufgenommen, daß der C-Bogen 1 entlang seines Umfanges um seine Mittelachse M verstellt werden kann. Dies kann in nicht dargestellter, an sich bekannter Weise manuell oder motorisch erfolgen. Der Halteschuh 7 ist an einem Tragarm 8 angebracht, der auf einer Führungsstange 9 längsverschieblich aber unverdrehbar angeordnet ist. Die Verschiebung des Tragarmes 8 auf der an einem nicht dargestellten ortsfesten Sockel angebrachten Führungsstange 9 kann in nicht dargestellter, an sich bekannter Weise manuell oder motorisch erfolgen. Außerdem weist das erfindungsgemäße Gerät eine Quelle fokussierter akustischer Wellen auf, bei der es sich um eine Druckimpulsquelle, und zwar eine Stoßwellenquelle 10, handelt. Als Stoßwellenquelle 10 ist vorzugsweise eine elektromagnetische Stoßwellenquelle vorgesehen, wie sie beispielsweise in der US-PS 4 674 505 oder in der DE-OS 33 12 014 beschrieben ist. Die Stoßwellenquelle 10 weist eine Fokuszone F auf, in der die erzeugten Stoßwellen zusammenlaufen und die auf der akustischen Achse A der Stoßwellenquelle 10 liegt. Die Stoßwellenquelle 10 ist derart an einem auf dem inneren Flansch des C-Bogens 1 geführten und entlang des Umfanges des C-Bogens 1 verstellbaren Wagen 11 angebracht, daß ihre akustische Achse A unabhängig von der Position des Wagens 11 auf dem C-Bogen 1 die Mittelachse M des C-Bogens 1 in dem gleichen Punkt wie der Zentralstrahl Z des Röntgenstrahlenbündels schneidet. Außerdem ist vorgesehen, daß während der Behandlung eines Patienten bzw. eines Körperteiles mit Stoßwellen unabhängig von der Position des Wagens 11 auf dem C-Bogen 1 die Mittelachse M des C-Bogens 1 durch das Zentrum der Fokuszone F verläuft. Die Verstellung der Stoßwellenquelle 10 längs des Umfanges des C-Bogens 1 erfolgt zwischen zwei durch den Röntgenstrahler 2 und den Röntgenbildverstärker 3 begrenzten Endpositionen, für die in der Figur jeweils die Lage der akustischen Achse A' bzw. A'' eingetragen ist, in nicht dargestellter, an sich bekannter Weise entweder manuell oder motorisch. Die Stoßwellenquelle 10 ist somit entlang des Umfanges des C-Bogens 1 um insgesamt einen Winkel "alpha" verstellbar.

Der C-Bogen 1 erstreckt sich über einen Winkel von mehr als 180°, und zwar dermaßen, daß der C-Bogen 1 entlang seines Umfanges in dem Halteschuh 7 um einen Winkel "beta" verstellt werden kann, der wenigstens der Differenz von 360° und dem Winkel "alpha" entspricht. Durch Verstellen des C-Bogens 1 entlang seines Umfanges in dem Halteschuh 7 und Verstellen der Stoßwellenquelle 10 mit ihrem Wagen 11 längs des Umfanges des C-Bogens 1 ist es also möglich, die Stoßwellenquelle 10 auf einer vollständigen Kreisbahn um die Mittelachse M des C-Bogens 1 herumzuführen. Dies ist, wie im folgenden deutlich werden wird, insbesondere für die Behandlung von Knochenleiden im Bereich der Extremitäten von Vorteil. Allerdings muß dazu der C-Bogen 1 um einen Winkel "epsilon", der wenigstens gleich dem Winkel "delta" ist, über den der Halteschuh 7 des C-Bogens 1 umgreift, länger ausgeführt sein als der Winkel "beta".

Soll ein Knochenleiden im Bereich einer Extremität, beispielsweise die Fraktur eines Oberarmes 12, behandelt werden, wird der Patient auf einem nicht dargestellten Operationstisch derart gelagert, daß die Mittelachse des Oberarmknochens in etwa mit der Mittelachse M des C-Bogens 1 zusammenfällt, wenn der Oberarm in einem Winkel von 90° von dem Körper des Patienten abgespreizt ist. Der Oberarm 12 und der Oberarmknochen 13 sind in der Figur schematisch im Querschnitt angedeutet. Der Oberarm 12 wird dann mittels in der Orthopädie bekannter Maßnahmen in der genannten Position zunächst fixiert. Die Lagerung sollte derart erfolgen, daß der Oberarm 12 im Bereich der Fraktur über seinen gesamten Umfang frei zugänglich ist. Nun wird die Röntgen-Ortungseinrichtung aktiviert und der Tragarm 8 längs der Führung 9 derart verstellt, daß sich die Fraktur etwa im Zentrum des auf dem Fernsehmonitor 5 sichtbaren Röntgenbildes befindet. Dann werden durch vorsichtiges Verlagern des Oberarmes 12 in einer Richtung, die etwa quer sowohl zur Mittelachse M des C-Bogens 1 als auch zum Zentralstrahl Z des Röntgenstrahlenbündels der Röntgen-Ortungseinrichtung verläuft, und eventuell durch Verschieben des Tragarmes 8 längs der Führung 9 der Oberarm 12 und der C-Bogen 1 relativ zueinander derart ausgerichtet, daß sich die Marke T mit demjenigen Bereich der Fraktur deckt, der zunächst mit Stoßwellen beaufschlagt werden soll. Ist dies der Fall, wird der C-Bogen um einen definierten Winkel "gamma", vorzugsweise gamma = 0,5 x (180 - alpha), verschwenkt und der Oberarm 12 erforderlichenfalls vorsichtig in derjenigen Richtung verlagert, die der Richtung des Zentralstrahles Z vor dem Verschwenken des C-Bogens um den Winkel "gamma" entspricht, bis sich die Marke T wieder mit demjenigen Bereich deckt, der mit Stoßwellen beaufschlagt werden soll.

Der mit Stoßwellen zu beaufschlagende Bereich befindet sich nun im Schnittpunkt der Mittelachse M des C-Bogens 1 mit dem Zentralstrahl Z und der akustischen Achse A. Die Stoßwellenquelle 10, die mittels einer rohrartigen Führung 14 teleskopartig längs ihrer akustischen Achse A verstellbar mit dem Wagen 11 verbunden ist und sich zuvor aus Gründen der besseren Zugänglichkeit in ihrer in der Figur strichliert angedeuteten Parkposition befand, wird nun in ihre in der Figur mit ausgezogenen Linien dargestelle Arbeitsposition gebracht, in der die Fokuszone F der Stoßwellen die beschriebene Position im Schnittpunkt der akustischen Achse A der Mittelachse M des C-Bogens 1 und des Zentralstrahles Z einnimmt. Dabei wird die Stoßwellenquelle 10 mit ihrem flexiblen Koppelkissen 15, das der akustischen Ankoppelung an das Jeweils zu behandelnde Körperteil dient, an die Oberfläche des Oberarmes 12 angepreßt. Dabei wurde zuvor der Wagen 11 längs des Umfanges des C-Bogens 1 in diejenige Position verfahren, in der die akustische Achse A der Stoßwellenquelle 10 unter einem dem dem Stoßwellen zu beaufschlagenden Bereich der Fraktur entsprechend gewählten Winkel zu dem Oberarm 12 bzw. dem Oberarmknochen 13 verläuft.

Da der C-Bogen 1 entlang seines Umfanges in dem Halteschuh 7 und der Wagen 11 mit der Stoßwellenquelle 10 entlang des Umfanges des C-Bogens 1 unabhängig voneinander verstellbar sind, besteht nun die Möglichkeit, nach erfolgter Ankoppelung der Stoßwellenquelle 10 an den Oberarm 12 zu überprüfen, ob der Oberarmknochen 13 noch in der erforderlichen Weise relativ zu der Fokuszone F der Stoßwellenquelle 10 ausgerichtet ist. Dies geschieht, indem zunächst bei derjenigen Stellung des C-Bogens 1, die dieser bei Vornahme der Ankoppelung der Stoßwellenquelle 10 an den Oberarm 12 einnimmt, mittels der Röntgen-Ortungseinrichtung die Ausrichtung des Oberarmes relativ zu der Stoßwellenquelle 10 überprüft wird. Anschließend wird der C-Bogen 1 unter Beibehaltung der Stellung der Stoßwellenquelle 10 relativ zu dem Oberarm 12 um den Winkel "gamma" in seine Ausgangslage verschwenkt worauf eine nochmalige Überprüfung der Lage des zu behandelnden Bereiches der Fraktur relativ zu der Fokuszone F der Stoßwellenquelle 10 erfolgt. Falls bei der Ankoppelung der Stoßwellenquelle 10 trotz der Fixierung des Oberarmes 12 Verschiebungen aufgetreten sind, können diese korrigiert werden. Es versteht sich, daß bei der Verstellung des C-Bogens 1 um den Winkel "gamma" eine Relativbewegung zwischen dem Wagen 11 und dem C-Bogen 1 stattfindet, um zu gewährleisten, daß der Stoßwellenquelle 10 ihre räumliche Lage beibehält. Falls sowohl der C-Bogen 1 in dem Halteschuh 7 als auch der Wagen 11 auf dem C-Bogen 1 motorisch verstellbar sind, läßt sich eine Verstellung des C-Bogens 1 bei gleichzeitiger Beibehaltung der räumlichen Ausrichtung der Stoßwellenquelle 10 leicht realisieren, indem die jeweiligen Antriebsmotore derart angesteuert werden, daß der C-Bogen 1 in dem Halteschuh 7 und der Wagen 11 auf dem C-Bogen 1 Bewegungen mit gleicher, jedoch entgegengesetzter Winkelgeschwindigkeit ausführen.

Ist sichergestellt, daß sich der zu behandelnde Bereich der Fraktur in der erforderlichen Weise in der Fokuszone F der Stoßwellenquelle 10 befindet, wird der im Fokus F der Stoßwellen befindliche Bereich des Oberarmknochens 13 mit einer Serie von Stoßwellen beaufschlagt, wodurch eine Anregung des Knochenwachstums bewirkt wird. Sollen weitere Punkte der Fraktur mit Stoßwellen beaufschlagt werden, so geschieht dies, indem in der zuvor beschriebenen Weise der jeweils zu behandelnde Punkt in den Schnittpunkt der Mittelachse M des C-Bogens 1 mit dem Zentralstrahl Z und der akustischen Achse A gebracht wird und die Stoßwellenquelle 10 zur Ankoppelung an den Oberarm 12 in eine solche Position gebracht wird, daß ihre akustische Achse A unter dem gewünschten Winkel relativ zu dem Oberarmknochen 12 verläuft. Dabei sind infolge des Umstandes, daß die Stoßwellenquelle 10, wie bereits erläutert wurde, auf einer vollständigen Kreisbahn um die Mittelachse M des C-Bogens 1 herumgeführt werden kann, beliebige Winkelstellungen möglich.

In diesem Zusammenhang kann es zweckmäßig sein, wenn der Röntgenstrahler 2 und der Röntgenbildverstärker 3 ihre Position tauschen können, so daß bezogen auf die Darstellung in der Figur wahlweise wie dargestellt der Röntgenstrahler 2 oben und der Röntgenbildverstärker 3 unten oder aber in nicht dargestellter Weise der Röntgenstrahler 2 unten und der Röntgenbildverstärker oben positioniert ist. Dies wird dadurch erreicht, daß der Tragarm 8 mit einem Gelenk 16 versehen ist, das - motorisch oder manuell - eine Schwenkung des C-Bogens 1 um eine rechtwinklig zur Mittelachse M stehende und diese in dem gleichen Punkt wie der Zentralstrahl Z und die akustische Achse A schneidende Achse X erlaubt, und zwar um ± 180°.

Anders als im Falle des beschriebenen Ausführungsbeispieles müssen der Schnittpunkt der akustischen Achse A mit der Mittelachse M und der Schnittpunkt des Zentralstrahles Z mit der Mittelachse M nicht notwendigerweise identisch sein. Ein eventueller Versatz läßt sich durch eine entsprechende Korrektur der Lage der Marke T korrigieren.

Obwohl im Falle des Ausführungsbeispieles die Erfindung im Zusammenhang mit einem Gerät zur Behandlung von Knochenleiden beschrieben ist, sind beliebige andere medizinische Anwendungen der Erfindung möglich.

Bei der Quelle akustischer Wellen muß es sich nicht notwendigerweise wie im Falle des Ausführungsbeispieles um eine Stoßwellenquelle handeln. Es kann vielmehr auch eine Druckimpulsquelle, die Zugimpulse (Unterdruck) aussendet, oder eine therapeutische Ultraschallquelle vorgesehen sein. Die Verwendung derartiger Quellen akustischer Wellen ist beispielsweise bei der Tumorbehandlung zweckmäßig.

**Patentansprüche**

1. Gerät zur Behandlung eines Lebewesens mit akustischen Wellen, aufweisend eine Röntgen-Ortungseinrichtung mit einem Röntgenstrahler (2) und einem Strahlenempfänger (3), welche einander gegenüberliegend an einem C-Bogens (1) angebracht sind, der entlang seines Umfanges um seine Mittelachse (M) verstellbar in einem Halter (7) aufgenommen ist, und eine Quelle (10) akustischer Wellen, die derart entlang des Umfanges des C-Bogens (1) verstellbar ist, daß die akustische Achse (A) der Quelle (10) akustischer Wellen stets die Mittelachse (M) des C-Bogens (1) schneidet.

2. Gerät nach Anspruch 1, **dadurch gekennzeichnet,** daß die Summe desjenigen Winkels ("beta"), um den der C-Bogen (1) entlang seines Umfanges in dem Halter (7) verstellbar ist, und desjenigen Winkels ("alpha"), um den die Quelle (10) akustischer Wellen entlang des Umfanges des C-Bogens (1) verstellbar ist, wenigstens gleich 360° ist.

3. Gerät nach Anspruch 1 oder 2, **dadurch gekennzeichnet,** daß die Quelle (10) akustischer Wellen fokussierte akustische Wellen abgibt, welche in einer auf der akustischen Achse (A) der Quelle (10) akustischer Wellen liegenden Fokuszone (F) zusammenlaufen, und daß die Fokuszone (F) der akustischen Wellen wenigstens während der Beaufschlagung eines zu behandelnden Bereiches mit akustischen Wellen auf der Mittelachse (M) des C-Bogens (1) liegt.

4. Gerät nach einem der Ansprüche 1 bis 3, **dadurch gekennzeichnet,** daß der C-Bogen (1) um eine Achse (X), die die Mittelachse (M) des C-Bogens (1) schneidet, um einen Winkel von wenigstens 180° schwenkbar ist.

**Europäisches Patentamt**

**EUROPÄISCHER RECHERCHENBERICHT**

Nummer der Anmeldung

EP    92 11 5001

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | WO-A-9 005 492 (MEDAS SOCIETA PER AZIONI)<br>* Seite 4, Zeile 17 - Seite 6, Zeile 27; Abbildungen 1-3 *<br>--- | 1-4 | A61B17/22<br>A61B6/00<br>A61B6/12 |
| Y | GB-A-2 120 060 (GRADY)<br>* Zusammenfassung; Abbildung 1 *<br>--- | 1-4 | |
| A | EP-A-0 277 489 (SIEMENS)<br>* Spalte 1, Zeile 54 - Spalte 2, Zeile 8; Abbildung 1 *<br>--- | 1 | |
| A | EP-A-0 169 311 (DORNIER)<br>* Seite 9, Zeile 20 - Seite 10, Zeile 13; Abbildung 2 *<br>--- | 1 | |
| A | EP-A-0 405 282 (SIEMENS)<br>* Spalte 10, Zeile 11 - Zeile 31 *<br>* Spalte 17, Zeile 3 - Zeile 28; Abbildung 9 * | 1 | |
| | ----- | | **RECHERCHIERTE SACHGEBIETE (Int. Cl.5)**<br><br>A61B |

Der vorliegende Recherchenbericht wurde für alle Patentansprüche erstellt

| Recherchenort | Abschlußdatum der Recherche | Prüfer |
|---|---|---|
| DEN HAAG | 17 DEZEMBER 1992 | MOERS R. |